# EUROPEAN PATENT APPLICATION

(11) **EP 4 489 016 A1**
(43) Date of publication of application: **08.01.2025**
(21) Application number: 23183363.3
(22) Date of filing: 04.07.2023
(51) Int. Cl.: G16H 20/40, A61C 7/00

(54) **METHOD FOR IMPROVING A TREATMENT PLAN FOR A DENTAL ALIGNER**

(71) Applicant: K Line Europe GmbH, 40589 Düsseldorf (DE)
(72) Inventor: Kandil, Sherif, 40474 Düsseldorf (DE)
(74) Representative: Kalkoff & Partner Patentanwälte mbB

(57) **Abstract**

The invention relates to a method for improving a treatment plan for a dental aligner and to an assembly. In order to an optimized treatment plan for a dental aligner, it is provided, that the method has the following steps:
- Gathering basic information regarding the patient;
- Determining and entering the initial position and a final target position of the dental aligner into a control unit;
- Using the control unit to calculate at least a first intermediate target position and at least a first stimulus to act on the dental aligner for a first correction step;
- Adapting the position of the dental aligner before reaching or upon achieving the first intermediate target position by using the basic information regarding the patient and updated basic and additional information regarding the patient, which has been entered into the control unit, said control unit calculating an updated first intermediate position and an updated second stimulus for a second correction step and
- Submitting said dental aligner to the updated second stimulus; wherein
- the control unit is designed to store the basic, the updated basic and the additional information and to calculate the intermediate, the updated intermediate target position and the final target position and the and updated stimulus;
- said basic, updated basic and additional information comprising information about the person to be treated other than information about the position of the dental aligner.

## Description

The invention relates to a method for improving a treatment plan for a dental aligner and to an assembly.

Dental aligners can be used to correct misaligned teeth. They are aids that are used in the mouth or on the teeth. Dental aligners typically consist of a polymer or a combination of polymers or of a metal, or a combination or alloy of metals, that change shape in a precalculated manner under the influence of a stimulus, or they comprise such material. The treatment of misaligned teeth requires an incremental change of teeth positions. This incremental change must take place precisely.

A method to calculate a stimulus of a medical aid that changes shape and therefore the position of a tooth from an actual position to a final target position is known, for example, from WO 2021/048318 A1. According to the current prior art in the case of a tooth correction, starting from the actual tooth position, a medical aid, here a tooth correcting device, is designed that undergoes a first change of shape under the influence of a first stimulus and then effects a first change of the tooth position in a first correction step. Because the actual position of a tooth to be corrected often does not match with the expected target position after the first correction step, a control unit calculates a stimulus that has to act on the medical aid to perform a second correction step, taking into account a deviation between the actual state and the target state of the tooth to be corrected. However, this does not yet allow for an optimal treatment for the patient.

It is an object of the invention to achieve an optimized treatment plan for a dental aligner.

This object is solved with a method according to claim 1 as well as with an assembly according to claim 14.

The method according to the invention for improving a treatment plan for a dental aligner has the following steps:
- Gathering basic information regarding the patient;
- Determining the initial position of the dental aligner;
- Entering the initial position of the dental aligner to be re-positioned into a control unit;
- Determining a final target position based on the gathered basic information and the initial position of the dental aligner;
- Entering the final target position of the dental aligner into the control unit;
- Using the control unit to calculate at least a first intermediate target position and at least a first stimulus to act on the dental aligner for a first correction step, said calculation taking into account the deviation between the initial position and final target position of the dental aligner;
- Adapting the position of the dental aligner before reaching or upon achieving the first intermediate target position by using the basic information regarding the patient and updated basic and additional information regarding the patient, which has been entered into the control unit, said control unit calculating an updated second intermediate target position and an updated second stimulus for a second correction step and
- Submitting said dental aligner to the updated second stimulus; wherein
- the control unit is designed to store the basic, the updated basic and the additional information and to calculate the intermediate, the updated intermediate target position and the final position and the first and updated stimulus;
- said additional and updated information comprising information about the person to be treated other than information about the position of the dental aligner.

Gathering of the basic and/or updated basic and/or additional information regarding the treatment plan for a dental aligner can take place in an analog manner, for example with a questionnaire, preferably however, the basic information is gathered digitally, for example by receiving real-time data and/or by retrieving and analyzing stored data from a storage device, for example a database. Improving the treatment plan, especially the different correction steps, is preferably achieved by continuous biological optimization of the treatment plan.

Basic and /or additional information preferably describe information that is known when the final target position is determined and/or the first or any other subsequent intermediate target positions and/or the first stimulus or any other following stimuli are calculated. The updated basic information and/or the additional information are preferably information that change throughout the treatment plan or were not known at the beginning of the treatment. For example, additional information are different values of the basic information. Preferably, additional information is already taken into account while determining the final and intermediate target positions. This for example allows the physician to account for planned holidays, scheduled flights or any other event which can be planned for in advance.

Determining the initial position of the dental aligner can take place conventionally in an analog manner, for example with an impression of the dental aligner; preferably, however, the initial position is detected by digital measurement, for example by means of digital photography. Alternatively, however, the initial position of the dental aligner is detected by digitally measuring the initial position of the dental aligner, or by signals from a sensor that detects the initial position of the dental aligner. Preferably, determining the initial position of the dental aligner takes into account an actual position of a tooth to be corrected. The initial position of the dental aligner and/or the actual position of the tooth to be corrected can also be detected by a sensor that reports the current position of the dental aligner and/or the tooth to be corrected. In an advantageous embodiment of the invention, the sensor can be arranged on the dental aligner, but the sensor can also be arranged on a scanner, for example, a digital intraoral scanner.

The analogically or digitally determined initial position of the dental aligner to be re-positioned is then entered into a control unit. The input can be done manually by entering the data into the control unit. If the data is present in digital form, the input can be carried out directly, preferably in an automated manner, for example, by upload-ing or transferring the data to the control unit. Advantageously, the input takes place at an input portal, in particular an input portal of the manufacturer of the dental aligner or an input portal of the attending physician, for example a screen or a reader that can record data for a three-dimensional position description of the dental aligner and/or a tooth. The input portal transmits the inputs to the control unit. The input portal can be displayed on a display, it can be displayed by means of application software (app) on a smartphone, but it can also be specially configured as a separate device with the sole purpose of making inputs about the initial position of the dental aligner and/or the actual position of the tooth to be corrected.

The basic, updated basic and additional information comprise information and/or data about the person to be treated other than information about the position of the dental aligner and/or the position about the tooth to be corrected with the dental aligner. Including updated basic information and additional information about the patient in the calculation of the intermediate, updated intermediate or final target positions and the stimuli or updated stimuli allows additional and improved adaptation of the treatment plan and therefore an optimized individual treatment for each patient throughout the whole treatment.

In an advantageous embodiment of the invention, the basic and/or the updated basic and/or the additional information comprises patient's lifestyle data and/or patients' biological data. Optionally, the basic information and/or the updated basic and /or the additional information can be entered into the input portal in order to incorporate the patient's lifestyle and/or the patient's biological data into the optimization of the treatment plan for the dental aligner.

The patient's lifestyle data preferably includes at least one information or any combination of information from the group of the patient's wearing behavior with regard to the dental aligner, behavior patterns of the use of dental aligner by the patient, his willingness to follow the treatment rules, the treatment progress, shift working, working rhythm, sleep rhythm, rise and sleep time, lack of sleep, travel schedule, sports activities, exercise, exercise duration and intensity, holidays, speeches, flights, financial data, insurance data, alcohol or drug consumption, recreational or festive events, general feeling.

Preferably, the patient's biological data includes at least one information or any combination of information from the group of one or a plurality of information relating to vital signs, blood test results, information about saliva composition and quality and/or quantity, hormone status, or other analysis data, for example regarding excretions of the patient, medical procedures, medical history, heart function, heart rate, blood pressure, information about past, present and intended diagnoses, illness or medical treatment, sensitivity to pain [algesia], allergies, body mass index, weight, body fat, oxygen saturation, body temperature, growth, ECG readings, implants, medication, blood oxygen levels, sleep apnoea, snoring, periodic cycle for women, teeth grinding, bruxism, periods with increased or reduced bone activity, e.g. illnesses, accidents or therapy.

Preferably, the basic information, the updated basic information and/or the additional information is input into the input portal in the way that can be easily processed by an algorithm or software, for example in assessment levels such as "low" - "medium" - "high" or in an assessment according to ratings, i.e., 1 to 5. It is preferred that the input portal displays the assessment levels for these inputs, so that the patient or the attending physician can quickly and easily select and input. Data that is detected, for example, by sensors, for example relating to the time periods in which the patient wears or uses the dental aligner or relating to the manner in which the patient wears or uses the dental aligner, can also be gathered. Data relating to the patient's time schedules, such as a travel or sleep schedule, or specific events, for example a wedding or a party, can also be captured and/or stored in the storage device. Capturing and/or storing of the data can be done randomly, in set time intervals or in real-time.

The control unit is designed to process software or algorithms. For simplification, calculations that take place by means of software, or respectively the processing of algorithms in the control unit, are described below as activity or actuation of the control unit. Furthermore, the control unit is designed to store the basic, updated basic and/or the additional information in a storage device and to calculate the first intermediate, the updated second intermediate position and the final target position and the first and updated stimulus.

A final target position for the dental aligner is determined based on the gathered basic information or a combination of one or more of the basic information, the updated basic information and/or the additional information, and the initial position of the dental aligner, said final target position being usually at a maximum deviation from the initial position of the dental aligner. The final target position may for example be determined in an analog manner, for example by a physician or in digital manner, for example by the control unit. The final target position of the dental aligner is then entered into the control unit and/or preferably stored in the storage device of the control unit. After the final target position of the dental aligner has been entered, the control unit calculates at least a first intermediate target position and at least a first stimulus to act on the dental aligner, said calculation taking into account the deviation between the initial position and final target position of the dental aligner.

Before reaching or upon achieving the first intermediate target position, the position of the dental aligner is adapted by using the basic information regarding the patient, the updated basic information and/or the additional information regarding the patient, which has been entered into the control unit. Adapting the position of the dental aligner before reaching the first intermediate target position is for example necessary if the aligner does not adjust as planned, e.g., will achieve the first intermediate target position slower than expected. The control unit then calculates an updated second intermediate position and an updated second stimulus. The dental aligner is then submitted to the updated second stimulus. The updated second stimulus is preferably calculated by taking into account a comparison between the current actual position of the dental aligner and/or tooth to be corrected and the intermediate target position of the dental aligner and/or tooth to be corrected. Any deviation that is found between the actual intermediate position and the intermediate target position is captured by the control unit after the input. Preferably intermediate target positions and/or updated intermediate target positions for the dental aligner for individual correction steps of the treatment up to the final target position are saved in the control unit.

On the basis of the comparison of the actual intermediate position with the intermediate target position, the stimulus and/or updated stimulus is calculated that is necessary to cause the dental aligner to change shape in the manner necessary for the second correction step. Optionally, it is calculated which sections of the dental aligner must be subjected to a stimulus in order to achieve a change of shape. In doing so, any deviation that is found between the actual intermediate position from the intermediate target position of the dental aligner is taken into account: If, for example, the dental aligner moves farther than originally intended or if the patient cannot tolerate the rapid movement of the dental aligner, the stimulus for the second correction step can be weaker or a different stimulus with a weaker effect can be selected because only a smaller correction movement is necessary. If the dental aligner moves less than originally intended or the patient signals that he desires a faster movement of the dental aligner, a stronger stimulus or a different stimulus with a stronger effect can be used.

Optionally, the well-being of the patient or the inputs regarding the condition of the patient are taken into account in the calculation of the stimulus for the next correction step. The time periods during which the patient wears the dental aligner can also be incorporated into the calculation of the stimulus. Alternatively, a change of shape of the dental aligner can also be brought about in each case by a combination of two or more stimuli. The basic information, the updated basic information and the additional information regarding the patient are preferably taken into account in the calculation of the updated stimulus for each subsequent correction step up to the final target position. For example, if the patient has to spent a specific time abroad, for example work related or on a holiday, the updated stimulus for the next correction step can be weaker or left out completely. In this manner, the correction of the tooth can be achieved efficiently, meaning quickly and with low stress on the patient.

In general, the dental aligner can be produced from any material. Preferably, it comprises exclusively dynamic synthetic material. However, it can also advantageously be a dental aligner that consists of a combination of dynamic synthetic material and conventional, static synthetic material, wherein the shape of the static synthetic material cannot be changed by a stimulus (with the exception of mechanical force). Furthermore, synthetic material, meaning dynamic material or dynamic material in combination with static plastic material, can be used with another material, typically, for example, a plastic/metal combination. According to another alternative, the synthetic material can be combined with natural or synthetic rubber. Preferably, all the materials used contribute to aligning the tooth, i.e., transferring it to the final target position. The method according to the invention is carried out with a dental aligner that is produced using a dynamic synthetic material that changes shape in a calculable manner under the influence of a stimulus. The change of shape of the dental aligner effects a movement of tooth from an actual position to a target position.

Such a material, typically a thermoplastic material, consists, for example, of two different polymers that abut one another, preferably abut one another in layers. In this case, the first polymer does not react to movement-inducing stimuli, while the second polymer reacts to movement-inducing stimulation, in particular by a shape and/or volume change. Movement-inducing stimuli can be contact with moisture or liquid, in particular water and water-containing liquids, temperature, in particular heat and/or radiation. Such plastics are also referred to as shape memory polymers (SMPs). Alternatively, metals or shape memory alloys (SMAs) can also be used for the dental aligner.

Many stimulations act as a stimulus, each of which are tailored to the material properties of the dental aligner, to effect a desired deformation. A stimulus is selected in particular from the group comprising the parameters time, temperature, pH value, current or respectively electrical voltage, radiation, in particular infrared radiation, sound, illumination and liquid, e.g., water or solvent. Alternatively, the stimulus is selected from a combination of these parameters, for example, temperature and pH value, water and temperature, or time and temperature. However, a stimulus can also be mechanical force which brings about a change of shape of the dental aligner.

If the composition of the dynamic synthetic material or alternatively the amount of the second polymer and its absorption capacity for a stimulus is known, the magnitude of the form change of the synthetic material generated by the change of shape or volume can be calculated. The same applies to SMA.

Such thermoplastic material is offered, for example, by the company Stratasys Inc. in conjunction with the 4D Printing Project, along with information about which form change the contact with a stimulus or with various stimuli effects. Stratasys Inc. hereby implements a printing method in practice that was developed by Skylar Tibbits of the Self-Assembly Lab at Massachusetts Institute of Technology MIT, Boston, USA, and for which Autodesk Inc. has developed the software with which synthetic material, in particular dynamic synthetic material, can be built by means of a 3D printer to form a product that changes its outer shape in a predictable manner under the influence of a movement-inducing and thus form-changing stimulus.

The subsequent deformability of the dynamic synthetic material or an SMA that is induced by external stimuli can be used by producing a dental aligner at least partially out of SMP or SMA, which change shape in a precalculated manner when acted upon by a stimulation. The dental aligner preferably comprises a wall that abuts the surface of the tooth or teeth to be corrected. Due to the deformation, the wall of the dental aligner which deforms in a calculated manner, exerts force on the tooth which the wall abuts and moves the tooth as a result into the target position. The advantage of the method according to the invention is that a new dental aligner does not have to be created for each step, and the change of shape takes place in an optimal manner, taking into account the physical development of the patient as well as preferably the patient's lifestyle and biological data.

The information about the effect of the stimulus, meaning of the various parameters of the dental aligner mentioned above, on the respective material of the dental aligner are each made known by the manufacturer and are thus known when producing the dental aligner. This information is saved in the control unit and is incorporated into the software, or respectively the algorithm, that is used to calculate the first stimulus and/or updated stimulus necessary for the second correction step.

The control unit is preferably connected to a treatment device, which is designed to cause the calculated first stimulus and/or updated stimulus to act on the dental aligner. The calculated stimulus is output by the control unit and afterwards advantageously transmitted to the treatment device. The treatment device can be a device into which the dental aligner is inserted or that is placed onto or connected to the dental aligner. The treatment device is therefore, for example, configured as a container which - depending on the embodiment - can be tempered and/or filled with liquid and/or into which the radiation, for example, infrared radiation, can be introduced. The treatment device can also have a connector for connecting to the dental aligner and introduce a stimulus, in particular current or heat, into the dental aligner via the connection. The treatment device is optionally designed to subject parts of the dental aligner specified by the storage unit to a stimulus. The treatment device can also be designed to apply mechanical force in order to bring about a change of shape of the dental aligner, for example by bending or pressing individual sections of the dental aligner. However, the treatment device can alternatively be integrated into the dental aligner in order, for example, to use heat as a stimulus. Preferably, the treatment device is designed to apply two or more stimuli in combination. The treatment device is characterized in that it triggers the stimulus according to the specification of the control unit, which stimulus effects a change of shape of the dental aligner. The stimulus usually acts on the dental aligner for a specified duration. The change of shape of the dental aligner thus takes place in sections between (updated) intermediate target positions until a maximum change of shape of the dental aligner has been achieved at the final target position. This process of shape change can typically be repeated as often as the dental aligner can still be deformed by a stimulus, or until the final target position of the dental aligner is reached.

In an advantageous embodiment of the invention, the control unit is linked to an information gathering unit. The control unit is preferably linked to one or more information gathering units of the group of a smart watch, a smart phone, a smart scale, a blood pressure monitor, medical measuring devices, a sleep analyzer or a notebook. The information gathering unit is preferably build that the patient is able to enter basic information and/or updated basic information and/or additional information. Advantageously, the information gathering unit is built to gather the information automatically, for example with at least one sensor, such as a heart rate sensor, a pulse sensor or a distance sensor. The information gathering unit can be linked to the control unit at specific times, for example before the calculation of an updated intermediate target position. Preferably, the information gathering unit is linked to the control unit via a wireless connection, such as Bluetooth, WIFI or a mobile network, for example 5G. The information gathering unit advantageously enables the patient to share updated basic information and/or additional information with the physician in real time, so that the physician can easily adapt and/or improve the treatment plan for the dental aligner.

According to an embodiment of the method according to the invention, the control unit is used to calculate at least two intermediate target positions and at least two stimuli to act on the dental aligner, said calculation taking into account the deviation between the a prior intermediate target position and a subsequent intermediate target position of the dental aligner, wherein the position of the dental aligner is adapted upon achieving each intermediate target position by using the basic, updated basic and/or additional information regarding the patient to calculate the stimulus for the subsequent intermediate or final target positions.

In an advantageous embodiment of the invention, the control unit stores the temporal progression of the intermediate target positions of the dental aligner and takes into account the temporal progression of the intermediate target positions when calculating the updated stimulus for the updated intermediate target positions.

According to a preferred development of the method according to the invention, the control unit calculates, emits and stores the duration to achieve the intermediate target positions and/or the updated intermediate target positions, the size of the next stimulus and/or the updated stimulus, and/or the parameters selected for the stimulation.

The method according to the invention preferably comprises a separate data set for each dental aligner, which can be edited after entering an editing authorization in the input portal, for example, by entering a new actual position of the dental aligner and/or the tooth to be corrected. This ensures that, in each case, data is input about the associated dental aligner.

The invention further comprises an assembly for improving a treatment plan for a dental aligner, comprising a control unit having means for calculating a stimulus and/or an updated stimulus and a storage device for data. The means for calculating are designed to calculate and, if applicable, select the stimulus and/or the updated stimulus for the next correction step, wherein data about the effect of at least one stimulus, preferably, however, about multiple stimuli, and data about the intermediate, updated intermediate or final target position of the dental aligner and the basic, updated basic and additional information are contained in the storage device for the initial position and for each individual correction step. The control unit is connected to an input portal for entering the initial position of the dental aligner and the final target position of the dental aligner. The control unit is linked to the afore-mentioned information gathering unit. This preferably allows the physician to easily receive updated information from the patient.

The means for calculating comprise software that calculates the first stimulus for the first correction step from the initial position of the dental aligner and, if applicable, a deviation between the first intermediate target position and the actual intermediate position of the dental aligner and the effect of the respective at least one stimulus on the dental aligner. In doing so, the software can switch or choose the updated stimulus or a combination of stimuli for the second and or subsequent correction step, depending on the magnitude of the change of shape of the dental aligner to be effected in the second correction step to move the tooth to be corrected. In addition, the software can determine the intensity of the stimulus, meaning, for example, the level of the temperature, the duration over which the stimulus must act. The stimulus can be chosen depending on a chosen treatment device so that it is ensured that the treatment device can also apply the calculated stimulus to the dental aligner.

The calculated updated stimulus for the second correction step is then emitted. The updated stimulus is either printed or displayed on an output portal, for example, a screen. Alternatively, the calculated stimulus is transmitted to a treatment device and implemented there, i.e., the updated stimulus acts on the dental aligner.

Entering the initial position of the dental aligner and/or the current actual position of the tooth to be corrected takes place as described above via an input portal. Advantageously, the input portal is connected to the control unit; further advantageously, the input portal also serves as an output portal.

It is further preferred if the input portal can only be opened for an input with an access authorization. It is further preferred if an input for a specific correcting device can only take place if the specific data set for this correcting device is opened by means of a code. This ensures that the upcoming change of shape of the dental aligner takes place correctly, because erroneous incorrect operation is precluded after the access code has been input.

It is further preferred that the control unit and, if applicable, the treatment device are preset for the dental aligner such that, if parts of the control unit or software are missing or fail, the activation of the dental aligner by the stimulus takes place for a following correction step according to the original treatment plan, so that the treatment is not interrupted but can merely be resumed in a less optimized manner.

Details of the invention are explained below with regard to an exemplary embodiment. In the figures:
- Fig. 1: shows a process diagram of the schematic mapping of the input portal and treatment device
- Fig. 2a: shows a process diagram of the complete calculation of the updated stimulus
- Fig. 2b: shows a process diagram of the calculation of the difference between the actual intermediate position and intermediate target position of the dental aligner
- Fig. 3: shows carrying out the change of shape

In a first step of the method according to the invention for improving a treatment plan for a dental aligner, basic information regarding the patient is gathered. Then, the initial position of the dental aligner is determined and entered into a control unit of an assembly. A final target position is determined based on the gathered basic information and the initial position of the dental aligner and entered into the control unit as well.

A treatment plan is created in which the path of the dental aligner from the initial position to the final target position is divided into at least two correction steps, often into a sequence of multiple correction steps. Corresponding to the treatment plan a dental aligner is created. The material of the dental aligner, usually comprising SMP or SMA, changes its shape in a precalculated manner under the influence of a stimulus, typically from an intermediate target position to a subsequent updated intermediate target position. The type and intensity of the stimulus determine the magnitude of the change of shape.

The necessary data required to determine the initial position, the final target position of the dental aligner, the intermediate target positions according to the individual correction steps, and the type and intensity of at least one stimulus, preferably multiple stimuli, are saved in a control unit. The control unit comprises a storage device for this purpose. The control unit is further equipped with a processor and with software that, after completion of a first correction step, calculates an updated stimulus for triggering a change of shape of the dental aligner for the second or any subsequent correction step.

The control unit can be a separate device with an input portal, for example, a keyboard or a touchscreen mask, and advantageously with an output portal, for example, a screen display or a printer.

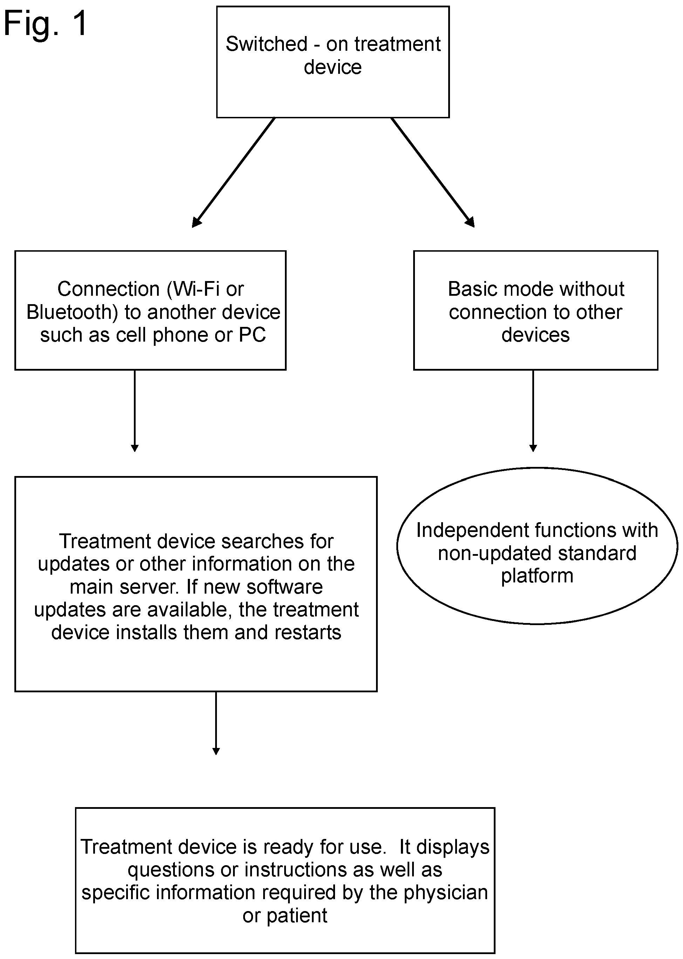

Preferably, the control unit is connected - as shown in Fig. 1 - to a computer, for example, in the form of a smartphone, a tablet, or a desktop computer. To make the connection to the control unit, a connection to the input portal, for example a separate application (app), can be downloaded, the input interface of which is connected to the input portal and the output interface of which is connected to the output portal of the control unit. The keyboard of the computer and the screen of the computer can be used for inputs into the input interface and thus into the input portal. Connecting the control unit to a computer has the advantage that the storage device and the software of the control unit can be kept constantly up to date through updates. Alternatively, a stand-alone control unit can be used.

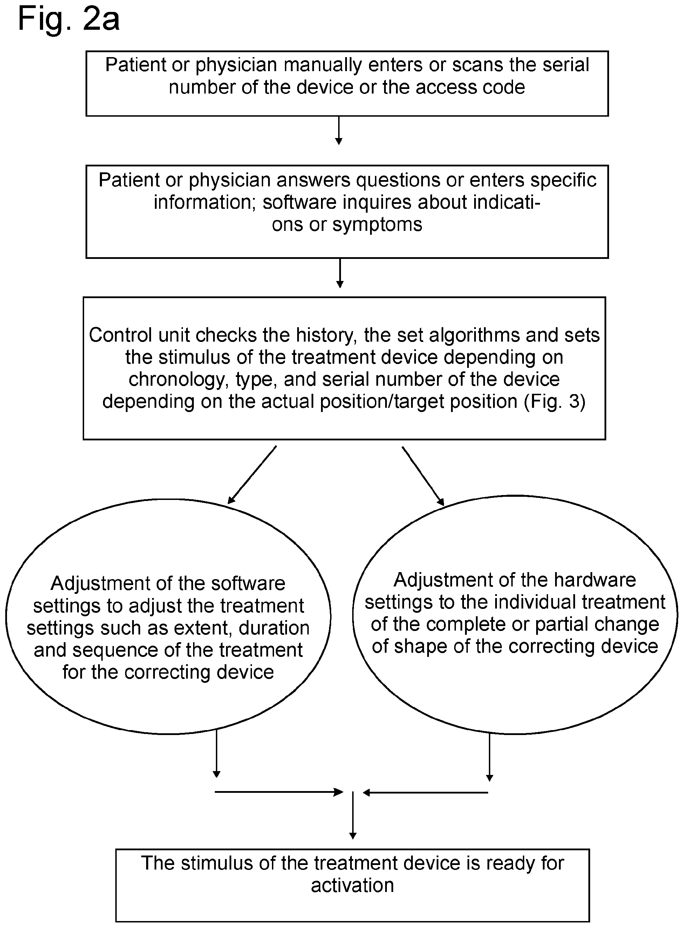

Starting from a control unit connected to a computer, after switching on and optionally loading any available updates, this control unit is ready for data input, either for creating a new file for a new dental aligner or for the evaluation of a correction step that has taken place and the calculation of an updated stimulus for a following correction step. The control unit or respectively the input portal can be designed for use by the patient, the attending physician, or the manufacturer of the dental aligner depending on who creates a new file for a dental aligner or evaluates a correction step and calculates the stimulus for a further correction step. Advantageously, according to Fig. 2a, an access authorization is provided for each user of the control unit to ensure that only authorized persons and, if applicable, users trained to use the control unit make inputs. It is also recommended to create a separate file with a separate access code for each dental aligner to ensure that a user who, for example, is overseeing multiple correcting devices as the attending physician always makes inputs for the correct dental aligner.

At the end of a first correction step, the change of shape of dental aligner and/or the tooth or teeth to be corrected that has been achieved so far is evaluated. The currently achieved actual intermediate position of the dental aligner and/or the actual position of the tooth to be corrected is detected, either manually, for example, through an impression and measuring the impression, or digitally by measuring and determining the actual intermediate position of the dental aligner and/or the actual position of the tooth to be corrected. The data obtained in this way is entered into the input portal of the control unit, either via a keyboard or by means of data transmission, for example, via a cable, Bluetooth, or a wireless network connection (WLAN, Wi-Fi).

Optionally, the input portal also asks the patient questions to gather basic, updated basic and/or additional information. The basic information, the updated basic information and the additional information comprise information about the patient's lifestyle and/or the patient's biological data and can be entered into the input portal in order to incorporate the patient's lifestyle and/or the patient's biological data into the optimization of the correcting tooth treatment by means of the dental aligner.

The patient's lifestyle data preferably includes at least one information or any combination of information from the group of the patient's wearing behavior with regard to the dental aligner, behavior patterns of the use of dental aligner by the patient, the willingness of the patient to follow the treatment rules, the treatment progress, shift working, working rhythm, sleep rhythm, rise and sleep time, lack of sleep, travel schedule, sports activities, exercise, exercise duration and intensity, holidays, speech habits, flights, financial data, insurance data, alcohol or drug consumption, scheduled events, general feeling.

Preferably, the patient's biological data includes at least one information or any combination of information from the group of one or a a plurality of information relating to vital signs, blood test results, information about saliva composition and quality and/or quantity, hormone status, or other analysis data, for example regarding excretions of the patient, medical procedures, medical history, heart function, heart rate, blood pressure, information about past, present and intended diagnoses, illness or medical treatment, sensitivity to pain [algesia], allergies, body mass index, weight, body fat, oxygen saturation, body temperature, growth, ECG readings, implants, medication blood oxygen levels, sleep apnoea, snoring, periodic cycle for women, teeth grinding, bruxism, periods with bone activity, e.g. illnesses, accidents or therapy.

Advantageously, standardized responses (for example: good - moderate - poor; high - medium - low) are provided which can be processed in an algorithm that evaluates the first correction step.

Optionally, the control unit is linked to one or more information gathering units of the group comprising a smart watch, a smart phone, a smart scale, a blood pressure monitor, medical measuring devices, a sleep analyser, a desktop computer and a tablet. The information gathering unit is preferably built such that the patient is able to enter basic information, updated basic information and/or additional information. Advantageously, the information gathering unit is built to gather the information automatically, for example with at least one sensor, such as a heart rate sensor, a pulse sensor or a distance sensor. The information gathering unit can be linked to the control unit at specific times, for example before the calculation of the updated intermediate target position or the updated stimulus for the next correction step. Alternatively, the information gathering unit is linked to the control unit via a wireless connection. The information gathering unit advantageously enables the patient to share updated basic or additional information with the physician in real time, so that the physician can easily adapt and/or improve the treatment plan for the dental aligner.

Data that is detected, for example, by sensors, e.g., from the information gathering unit, for example relating to the time periods in which the patient wears or uses the dental aligner or relating to the manner in which the patient wears or uses the dental aligner, can also be captured. Data relating to the patient's time schedules, such as a travel or sleep schedule, or specific events, for example a wedding or a party, can also be captured and/or stored in the storage device.

The patient can for example use the information gathering unit to enter information, e.g., with mobile apps. For example, an app tracking a woman's periodic cycle might be connected to an orthodontic treatment app which transmits the data to the control unit after receiving the approval of the patient. The data being conveyed to the orthodontic treatment app can be used to optimize the treatment plan and/or inform the dentist or orthodontist.

The basic, updated basic and additional information are taken into account when adapting the position of the dental aligner upon achieving the first intermediate target position and determining the stimulus required to achieve the at least second intermediate target position. For example, if the patient has to spent a specific time abroad, i.e., work related or on a holiday, the updated stimulus for the next correction step can set be weaker or left out completely.

For example, the periodic cycle of women influences the effect on the natural tooth mobility due to fluctuations on the hormone levels and a corresponding modification of the bone metabolism. Updated basic or additional information about phases of the periodic cycle, e.g., with varying degrees of pain sensitivity are taken into account when determining the stimulus. During a phase of high pain sensitivity, the updated stimulus for next correction step can be set lower. Alternatively, if the pain sensitivity is low, the updated stimulus for next correction step can be set with a higher force or longer duration to utilize the increased teeth mobility of the patient.

Furthermore, if a patient has a higher bone activity, e.g., because of an illness, therapy or an accident which involves the treatment of fractured bones, e.g., a jaw fracture, the updated stimulus for the next correction step or steps can be set higher and for longer durations. In this case the dental aligner can be changed faster with higher stimuli because the patient is taking pain killers and the bone metabolism is increased.

Other biology data that affect the teeth movement efficiency and speed and thus the change of the dental aligner are a lack of sleep, sleep apnea and snoring, teeth grinding and bruxism. The increase in the teeth movement efficiency and speed of this biology data allow to determine an updated stimulus with a higher force and/or longer duration for the next correction step. The patient's exercise intensity or blood oxygen levels also affect the teeth movement speed and accordingly the determination of an updated stimulus with a higher duration.

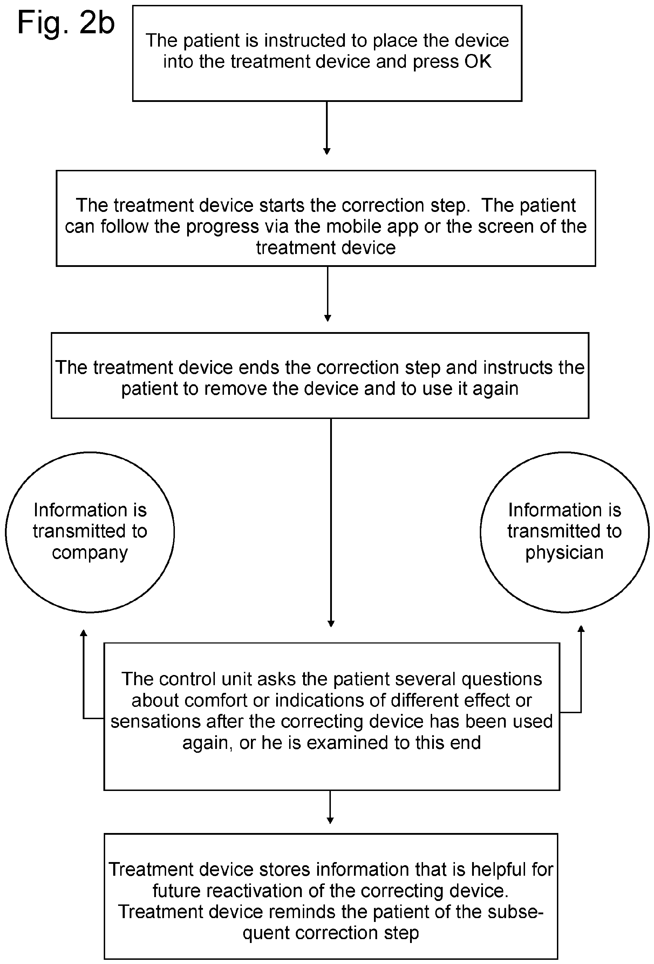

To calculate the updated stimulus that the change of shape of the dental aligner should effect for the following correction step, it is ascertained in accordance with Fig. 2b whether a deviation exists between the actual intermediate position and the calculated intermediate target position of the dental aligner and/or the tooth to be corrected that is provided or expected according to the treatment plan. Such a deviation is ascertained, for example, by means of measuring the dental aligner and/or tooth position with an intraoral scanner or by comparing digital photographs; it can, however, also take place by creating a current impression and comparing it to the previous impression that was taken before beginning the completed correction step.

If such a deviation exists, this is taken into account for the calculation of the updated stimulus. Furthermore, in accordance with Fig. 2a, the responses to the questions of the control unit (if such questions have been asked) and data received from the information gathering unit are taken into account for the calculation of the stimulus and/or the updated stimulus needed to achieve the second or subsequent intermediate or final target position. The control unit or respectively the algorithm also takes into account the settings specified in the original treatment plan for the stimulus that should act on the dental aligner at the beginning of the following correction step. The specified settings are now recalculated, taking into account any deviations of the actual intermediate position from the intermediate target position as well as the patients' responses, for example, about tolerability or intensity of the treatment, in order to move the dental aligner and/or the tooth to be corrected into the final target position as quickly as possible with minimal stress to the patient. If applicable, the control unit or respectively the algorithm chooses a different stimulus and/or a different intensity, for example, a switch from heat to liquid or to a combination of stimuli, for example heat and liquid, depending on the extent to which a change of shape of the dental aligner is necessary.

The method according to the invention thus ensures that correcting the tooth position does not take place according to a rigid treatment plan specified at the beginning of the treatment, but rather that the correction is carried out in an optimized manner, taking into account the actual course of the correction. This results in a short correcting treatment that is gentle for the patient.

The recalculation of the updated stimulus for the change of shape of the dental aligner is then emitted by the control unit via the output portal. According to a first alternative, the calculation of the stimulus is, for example, printed or displayed by the output portal and is manually transferred to a treatment device. The treatment device can be arranged directly on the dental aligner, for example, as a heat source. The treatment device is often configured as a container in which the dental aligner can be accommodated. The treatment device is further designed to allow at least one stimulus to act on the dental aligner; advantageously, two or more stimuli can act on the dental aligner, for example, temperature, liquid and radiation, each individually or in combination.

According to an alternative embodiment, the control unit is connected to and controls the treatment device. The connection can take place by installation in the housing of the treatment device, but it can also be made by a wired or wireless connection, in that the control unit sends actuation signals to the treatment device.

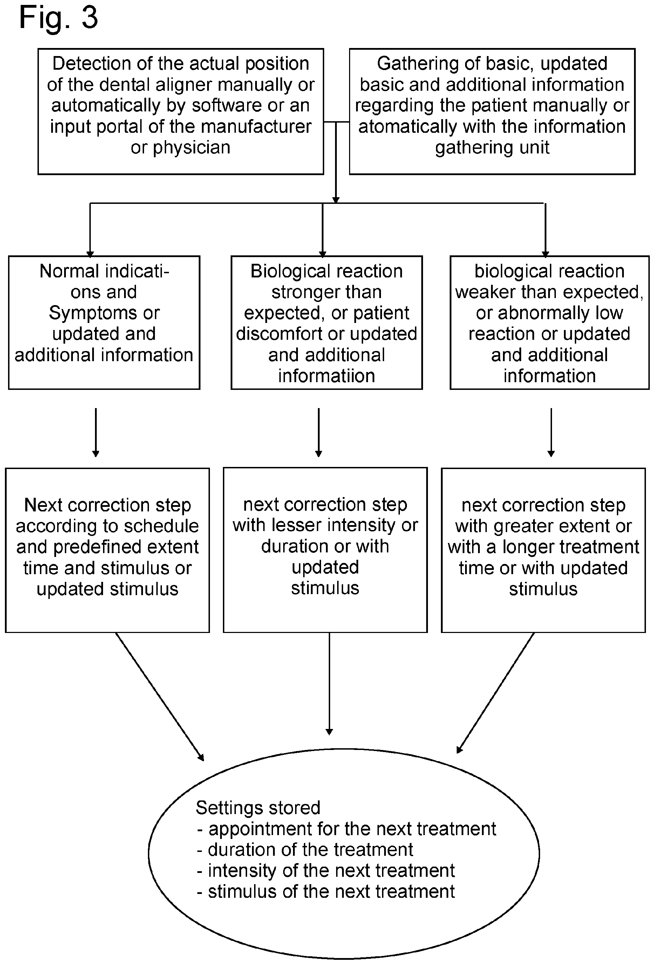

When controlling the treatment device in accordance with Fig. 3, the new calculation triggers the activation of one or more stimuli according to the specifications of the previous calculation of the control unit. If applicable, the control unit also causes the display of indicators in advance via the output portal, for example, the indicator to place the dental aligner into the treatment apparatus, or the indicator to switch on the treatment apparatus after placing the dental aligner and thus trigger the change of shape as a result of the action of the stimulus or the combined stimuli. Further optionally, the output portal displays the course of the action of the stimulus and the resulting change of shape of the dental aligner.

After completion of the application of the stimulus and/or the updated stimulus, the output portal optionally displays information about the subsequent correction step, for example, how long the subsequent correction step takes, how long the dental aligner is to be worn in each case in order to ensure optimal treatment success.

Optionally, after the dental aligner is inserted by the patient, the output portal then shows the patient some questions that relate in particular to the oral fit of the dental aligner, the shape of which has been changed, as well as questions about the sensations (or paresthesia) while the dental aligner is worn or questions about the effect of the dental aligner on the teeth to be corrected or other teeth. In this manner, it is ensured that any erroneous deformations of the dental aligner are discovered early.

As shown in Fig. 3, the control unit stores the data used to calculate the stimulus and/or the updated stimulus so that all information - preferably including the basic, updated basic and additional information regarding the patient- about the treatment is available at the end of the treatment. If applicable, this information is transmitted to the attending physician and/or the manufacturer of the dental aligner. If the treatment with the dental aligner comprises more than two correction steps, all the information available from previous correction steps can be used to calculate the stimulus for a subsequent correction step.

Finally, the manner in which the differences between the actual intermediate position of the dental aligner and the intermediate target position of the dental aligner ascertained in accordance with Fig. 2b are incorporated into the calculation of the stimulus for the change of shape of the dental aligner for the respective next correction step will be briefly explained.

If it is found after the completion of a first correction step that no deviation between the actual intermediate position and the intermediate target position exists, the calculation of the updated stimulus for the change of shape of the dental aligner takes place for the following correction step according to the original treatment plan or the updated stimulus is adapted by using the basic, the updated basic and the additional information only.

If the dental aligner and/or the tooth to be corrected has moved farther than expected or if the patient reports discomfort, then the treatment plan is changed for the next correction step. A less intense updated stimulus or a different, less intense updated stimulus is selected (for example, liquid instead of radiation) by the control unit or respectively by the algorithm.

If the dental aligner and/or the tooth to be corrected has moved less than expected or if the patient explicitly reports good health, then the treatment plan is changed for the next correction step. A more intense updated stimulus or a different, more intense updated stimulus is selected (for example, a longer action of the stimulus) by the control unit or respectively by the algorithm. A combination of stimuli can also be used, always within the scope of the stimuli that the respective treatment device can cause to act on the dental aligner.

It will be explained below how the control unit of the assembly or respectively the software required to implement the method according to the invention is created. For example, ATMEL chips can be provided with an access code that limit access to the control unit to authorized persons, since the chip can no longer be opened without the correct control code. The access code can be created, for example, with AVR Studio software. As the control unit, for example, an Espressif Systems Wi-Fi/BLESOC microchip (for example, esp8266 WIFI/esp32 WIFI/BLE) can be used that is based on a 32-bit microcontroller that has a sufficient number of digital I/O access points to control all electronic peripheral devices and analog inputs for detecting sensor signals and that also has sufficient storage capacity to store the software codes.

To create the software or respectively the algorithm, an Arduino IDE platform can be used, that is suitable for creating interactive software. The software is then transferred to a chip by means of a suitable printer. Later, series-produced printed circuit boards (PCBs) can be used, on which the software is stored and which serve as the control unit. If the control unit is installed in a treatment device, this can advantageously connect to a server on its own, for example, of the manufacturer and download updates. It is preferred that an input screen connected to the input portal and, if applicable, a display about the progress or status of the change of shape can be downloaded onto external computers such as smartphones, tablets, or desktop computers as an application (app).

The chip or respectively the circuit board on which the software is stored can now control numerous processes, including Wi-Fi or Bluetooth connections for transferring data from computers such as smartphones, tablets, or desktop computers, the input and the output portals, which, if applicable, are displayed on these computers, the management of storage devices on which data about the patient and/or about the dental aligner or treatment plan is stored, and, if applicable, also the treatment device, by which a stimulus acts on the dental aligner to prepare a following correction step. In this case, the chip or respectively the circuit board controls, for example, the switching on and off of the respective stimulus, for example, a heating, a cooling, a radiation source or the addition of liquid, both individually and in combination. Thus, for example, a change of shape of a dental aligner can require that first a specified amount of liquid, e.g., water, is let into a container containing the dental aligner. It must be indicated beforehand that the dental aligner has been correctly inserted into the container. This can be reported optically by a camera or by sensors or switches to the chip or the circuit board. Then, the water must first be heated and then cooled, wherein time specifications for the heating and the cooling must be complied with. Finally, it must be indicated that the change of shape is complete. Then, the data about the change of shape, both the information about the actual intermediate position and the intermediate target positions as well as the inputs about the basic, updated basic and additional information of the patient and the calculation of the stimulus and/or updated stimulus, must optionally be stored and, if applicable, transmitted to the physician and manufacturer of the dental aligner.

The control unit or respectively the chip or the circuit board can be brought into connection with a server of the physician or manufacturer, which server can be reached via a static IP address with a domain name. The control unit can retrieve updates or also modifications of the treatment device from the server. A Raspberry Pi server, for example, can be used, on which the operating system Raspbian, an Apache server, and contents programmed in PHP for web applications are installed.

In a second exemplary embodiment we will explain a possible embodiment of the method according to the invention using the control unit of the assembly according to the invention. A patient wears a first dental aligner for a week. According to the treatment plan, the dental aligner should go through two changes of shape, each after one week. After entering the access code, the control unit or respectively software according to the invention is designed to first ask the patient which dental aligner he is wearing and since when he has been wearing the dental aligner. After entering further data on the actual intermediate position of the dental aligner and/or the tooth to be corrected and updated basic and/or additional information, the updated stimulus necessary for the upcoming change of shape of the dental aligner is calculated. It is taken into account here that the updated stimulus for the second correction step, meaning at the beginning of the second week, is to be calculated. The correct stimulus is therefore calculated without error and the treatment device is controlled accordingly by the control unit. After the second week, the same procedure takes place, taking into account the updated timeline. The updated stimulus is calculated for the third correction step, meaning for the third week. If the patient erroneously attempts to trigger a change of shape of the dental aligner between the individual correction steps or after the completion of the correcting treatment, it is provided according to the present embodiment of the invention that the control unit displays that no change of shape is currently provided, or that the shape of the dental aligner is up to date. This prevents erroneously undesired changes of shape from being made to the dental aligner.

The assembly for improving a treatment plan for a dental aligner comprises the control unit connected to the input portal and one or more information gathering units.

## Claims

1. Method for improving a treatment plan for a dental aligner, comprising the steps of
- Gathering basic information regarding the patient;
- Determining the initial position of the dental aligner;
- Entering the initial position of the dental aligner to be re-positioned into a control unit;
- Determining a final target position based on the gathered basic information and the initial position of the dental aligner, said final target position being at a maximum deviation from the initial position of the dental aligner;
- Entering the final target position of the dental aligner into the control unit;
- Using the control unit to calculate at least a first intermediate target position and at least a first stimulus to act on the dental aligner for a first correction step, said calculation taking into account the deviation between the initial position and final target position of the dental aligner;
- Adapting the position of the dental aligner before reaching or upon achieving the first intermediate target position by using the basic information regarding the patient and updated basic and additional information regarding the patient, which has been entered into the control unit, said control unit calculating an updated second intermediate position and an updated second stimulus for a second correction step and
- Submitting said dental aligner to the updated second stimulus;
wherein
- the control unit is designed to store the basic the updated basic and additional information and to calculate the intermediate, the updated intermediate target position and the final target position and the first and updated stimulus;
- said basic, updated basic and additional information comprising information about the person to be treated other than information about a tooth to be corrected with the dental aligner.

2. Method according to claim 1, wherein the updated basic and/or the additional information comprises patient's lifestyle data and/or patients' biological data.

3. Method according to claim 2, wherein the patient's lifestyle data includes at least one information from the group of the patient's wearing behavior with regard to the dental aligner, behavior patterns of the use of dental aligner by the patient, the willingness of the patient to follow the treatment rules, the treatment progress, shift working, working rhythm, sleep rhythm, rise and sleep time, lack of sleep, travel schedule, sports activities, exercise, exercise duration and intensity, holidays, speech habits , flights, financial data, insurance data, alcohol or drug consumption, scheduled events, general feeling.

4. Method according to claim 2, wherein the patient's biological data includes at least one information from the group of one or a plurality of information relating to vital signs, blood test results, information about saliva composition and quality and/or quantity, hormone status, or other analysis data, for example regarding excretions of the patient, medical procedures, medical history, heart function, heart rate, blood pressure, information about past, present and intended diagnoses, illness or medical treatment, sensitivity to pain [algesia], allergies, body mass index, weight, body fat, oxygen saturation, body temperature, growth, ECG readings, implants, medication, blood oxygen levels, sleep apnoea, snoring, periodic cycle for women, teeth grinding, bruxism, periods with increased or reduced bone activity.

5. Method according to one of the preceding claims, wherein the control unit is linked to an information gathering unit.

6. Method according to one of the preceding claims, wherein the initial position of the dental aligner is detected by digitally measuring the initial position of the dental aligner, or by signals from a sensor that detects the initial position of the dental aligner.

7. Method according to one of the preceding claims, wherein determining the initial position of the dental aligner takes into account an actual position of a tooth to be corrected, wherein the actual position of the tooth to be corrected is detected by manual input into the control unit or by an input portal of the manufacturer of the dental aligner or by an input portal of the attending physician.

8. Method according to one of the preceding claims, wherein the control unit is connected to a treatment device, which is designed to cause the calculated stimulus and/or updated stimulus to act on the dental aligner.

9. Method according to one of the preceding claims, wherein the control unit is used to calculate at least two intermediate target positions and two stimuli to act on the dental aligner, said calculation taking into account the deviation between a prior intermediate target position and the subsequent intermediate target position of the dental aligner, wherein the position of the dental aligner is adapted upon achieving each intermediate target position by using the basic and additional information regarding the patient and the updated information regarding the patient to calculate the stimulus for the subsequent intermediate or final target position.

10. Method according to one of the preceding claims, wherein the control unit stores the temporal progression of the intermediate target positions of the dental aligner and takes into account the temporal progression of the intermediate target positions when calculating the updated stimulus for the next intermediate target positions.

11. Method according to one of the preceding claims, wherein the control unit calculates, emits and stores the duration of the intermediate target positions and/or the updated intermediate target positions, the size of the next stimulus and/or the updated stimulus, and/or the parameters selected for the stimulation.

12. Method according to one of the preceding claims, wherein a separate data set is created for each dental aligner, which data set can be edited after entering an editing authorization.

13. Method according to one of the preceding claims, wherein the basic and/or the updated basic and/or the additional information and/or the progression of the corrections steps is stored in the information gathering unit and/or the control unit.

14. Assembly for improving a treatment plan for a dental aligner, comprising a control unit for carrying out a method according to claim 1, connected to an input portal for entering the initial position of the dental aligner and the final target position of the dental aligner, and with means for calculating a stimulus for the change of shape of the dental aligner as well as with a storage device wherein the effect of the stimulus and the updated stimulus on the dental aligner is saved in the control unit, wherein the storage device of the control unit is designed to store the temporal progression of the correction steps, wherein the control unit is linked to an information gathering unit.
